(19)

**Europäisches Patentamt**
**European Patent Office**
**Office européen des brevets**

(11) **EP 1 518 568 B1**

(12) **EUROPEAN PATENT SPECIFICATION**

(45) Date of publication and mention
of the grant of the patent:
**27.05.2009 Bulletin 2009/22**

(51) Int Cl.:
*A61L 27/34* (2006.01)     *A61L 28/00* (2006.01)
*A61L 29/08* (2006.01)     *A61L 31/10* (2006.01)
*A61L 33/06* (2006.01)

(21) Application number: **03078031.6**

(22) Date of filing: **25.09.2003**

(54) **Complex coacervate core micelles as anti-fouling agents**

Mizellen mit einem Komplexkoazervatkern als fäulnishemmende Mittel

Micelles avec un noyau de complex coacervé comme agents antisalissures

(84) Designated Contracting States:
**AT BE BG CH CY CZ DE DK EE ES FI FR GB GR HU IE IT LI LU MC NL PT RO SE SI SK TR**

(43) Date of publication of application:
**30.03.2005 Bulletin 2005/13**

(73) Proprietors:
• **Wageningen Universiteit, Agrotechnologie en Voedingswetenschappen**
**6700 EV Wageningen (NL)**
• **RHODIA CHIMIE**
**92100 Boulogne Billancourt (FR)**

(72) Inventors:
• **Cohen Stuart, Martinus Abraham**
**6705 BK Wageningen (NL)**
• **Van der Burgh, Stefan**
**6708 HM Wageningen (NL)**
• **Fokkink, Reint Gerrit**
**7255 BW Hengelo (NL)**
• **De Keizer, Arie**
**6708 KX Wageningen (NL)**

(74) Representative: **Boittiaux, Vincent et al**
**Rhodia Services,**
**DPI,**
**40, rue de la Haie-Coq**
**93306 Aubervilliers Cedex (FR)**

(56) References cited:
WO-A-01/32230          WO-A-02/096477
US-A1- 2002 006 493          US-B1- 6 444 318

• KOVACEVIC D ET AL: "Specific ionic effects on weak polyelectrolyte multilayer formation" J PHYS CHEM B; JOURNAL OF PHYSICAL CHEMISTRY B AUG 14 2003, vol. 107, no. 32, 14 August 2003 (2003-08-14), pages 7998-8002, XP0009026233
• COHEN STUART MARTIEN A ET AL: "Kinetics of formation and dissolution of weak polyelectrolyte multilayers: Role of salt and free polyions" LANGMUIR; LANGMUIR JUL 9 2002, vol. 18, no. 14, 9 July 2002 (2002-07-09), pages 5607-5612, XP002270851
• EMOTO KAZUNORI ET AL: "Core-shell structured hydrogel thin layer on surfaces by lamination of a poly(ethylene glycol)-b-poly(D,L-lactide) micelle and polyallylamine" LANGMUIR; LANGMUIR JUN 2000 ACS, WASHINGTON, DC, USA, vol. 16, no. 13, June 2000 (2000-06), pages 5738-5742, XP0002172103

EP 1 518 568 B1

**Description**

Field of invention

The present invention relates to devices carrying on the surface polymeric micelles and a process for the preparation thereof. It further relates to the use of polymeric micelles as a surface coating for rendering a surface anti-fouling and/or protein-resistant.

Background

Many devices and materials require properties at the surface to be distinct from the bulk properties of the device or material, particularly in the case of biomedical devices. In order to make these devices biocompatible or prevent non-specific interaction of biopolymers such as plasma proteins and nucleic acids with the surface of the device, their surface properties are improved using coating methods.

Contact lenses are a good example of biomedical devices that require improved surface characteristics. In order to maintain good corneal health they are usually built up from hydrophobic materials that exhibit relatively high oxygen permeability through the bulk of the lens. However, without any surface treatment or modification such a lens will adhere to the eye. Thus a contact lens will generally have a core bulk material that is highly oxygen permeable and hydrophobic, and a surface that has been treated or coated to increase hydrophilic properties. This hydrophilic surface allows the lens to move relatively freely on the eye without adhering excessive amounts of tear lipid and protein.

For the above-mentioned purposes of rendering devices biocompatible, anti-fouling and/or protein non-adsorbing, it is known from the art to coat them with one or more layers of homopolymers, block polymers or mixtures thereof. It is preferred to apply block polymers with the desired hydrophobic and hydrophilic properties combined in one molecule. Coating of these macromolecules is realised either through grafting them onto the surface chemically or by adsorbing them from solution physically.

In WO A 01/32230 it is disclosed how to conveniently use the tendency of those kind of block polymers to self-assemble into regularly shaped micelles upon dissolution in a so-called selective solvent, which is a good solvent for one of the blocks, but a poor one for another. In polar media, such as water, block polymers with hydrophilic and hydrophobic segments aggregate in a way that the hydrophilic corona shields the hydrophobic core from the polar media. In non-polar media, the micelles are reversed. The shape and size of the micelle can be tuned by simply varying either the kind of monomer or the size and proportion of the constituting blocks. Coating of these polymeric micelles with either a hydrophobic core and a hydrophilic shell or the other way around according to WO A 01/32230 yields more defined and uniform layers at the surface than when applying the unassembled macromolecules.

However, the applicability of polymeric micelles with an amphiphilic character is restricted only to a limited number of combinations of surfaces and environment. Not only do the different constituents have to match the properties of the device and be able to yield its biocompatible, anti-fouling or protein non-adsorbing character, but geometry constraints are to be satisfied: self-assembly is induced only if the solvent selectivity among the different parts of the block polymers is sufficient.

Furthermore, like micelles prepared from low molecular weight surfactants, polymeric micelles only form above a so-called critical micelle concentration. Lower concentrations can destruct the micellar coating, which after all consists of physical rather than chemical associations of the amphiphilic molecules.

In WO A 01/32230 these problems are overcome by supplying block polymers with functional end groups through which the micelles chemically attach to the surface and by forming multilayers of micelles with other polymers or reversed micelles. The methods associated therewith are rather laborious, require additional synthesis steps and are still in need of a combination of distinct hydrophobic and hydrophilic blocks.

Description of the invention

It is the object of the invention to provide a coating composition comprising polymeric micelles that do not have the above-mentioned drawbacks of the selected group of amphiphilic polymeric micelles. The coating composition according to the invention can be applied to various types of surfaces, uses straightforwardly synthesised constituents and is not bothered by a CMC.

It is further an object of the invention to provide with a use of a coating composition comprising at least one polymeric micelle, wherein the polymeric micelle has a hydrophilic, neutral corona and a complex coacervate core, wherein the complex coacervate core is formed by charge complexation, for rendering at least one surface of a device protein-resistant.

The term "complex coacervation", as used herein, refers to the interaction of two macromolecules of opposite charge. In the literature coacervation addresses the separation of colloidal systems in liquid phases, wherein the phase more concentrated in hydrophilic colloid component is called the coacervate. The term "complex" indicates that the driving force for separation of colloids is of electrostatic origin. Micelles that are formed by the principle of complex coacervation are called complex coacervate core micelles (CCCM).

In the context of the polymeric micelles of the invention complex coacervation is sometimes otherwise referred to as complex flocculation, block ionomer complexation (BIC), polyelectrolyte complexation (PEC) or even interpolyelectrolyte complexation (IPEC).

The essential parts of the complex coacervate core micelles are a charged block, a second charged block with opposite charge, and a hydrophilic and neutral block. All constituents exhibit excellent water solubility.

In one embodiment of the invention these essential features are comprised in a single polymer, preferably a triblock terpolymer with an anionic block, a cationic block and a neutral hydrophilic block, in any order. However, in a preferred embodiment the polymeric micelle according to the invention comprises at least a first and a second polymer.

The first and the second polymer are oppositely charged, meaning that one polymer exhibits an overall anionic character and the other polymer has an overall cationic character. The ionic properties can be accounted for by groups that are permanently charged or "quenched" in aqueous environment, such as e.g. sulfonated groups, or by chargeable or "annealed" groups which are dependent on pH, such as e.g. amines and carboxylated groups. From hereon, the term "chargeable", as used herein, refers to both permanently charged groups and chargeable groups.

The first polymer is a block polymer with an ionic block comprising at least 6, more preferably at least 20 and most preferably at least 40 chargeable groups. The term "block polymer", as used herein, refers to polymers that are constructed from blocks of more than one monomer. It does not comprise polymers with random distributions of more than one monomer. According to the invention it is preferred that the block polymer is a diblock copolymer with end-to-end chains or a triblock terpolymer with a third block in-between.

The term "ionic block", as used herein, is meant to include blocks of charged groups and chargeable groups. The block polymer can be overall cationically or anionically chargeable. Preferably an ionic block with a sequence of at least 6, more preferably at least 20, and with most preference at least 40 chargeable groups can be employed for the polymeric micelles according to the invention.

The ionic block of the block polymer is preferably selected from the group consisting of poly-L-lysine or other poly(amino acids), polyacrylic acid (PAA), polymethacrylic acid (PMA), DNA-segments, poly(thiophene-3-acetic acid), poly(4-styrenesulfonic acid), polyvinylpyrrolidone, poly(pyridinium acetylene), poly(ethylene imine), poly(vinylbenzyltriamethylamine), polyaniline, polypyrrole, poly(alkylamine hydrochloride), poly-(dimethylamino ethylmethacrylate) (PAMA), polyaspartic acid, poly(N-alkyl-4-vinylpyridinium) (PVP), but it is not limited thereto. More preferably, the ionic block is selected from the group of polyacrylic acid (PAA), polymethacrylic acid (PMA), poly-(dimethylamino ethylmethacrylate) (PAMA) and poly(N-alkyl-4-vinylpyridinium) (PVP).

The first polymer further comprises at least a hydrophilic and neutral block. If the first polymer is a linear polymer, the blocks are end-to-end linked. If the first polymer is branched, the chargeable groups could be grafted onto a hydrophilic and neutral backbone or vice versa. However, it is preferred that the first polymer is a linear chain.

In principle any hydrophilic and neutral block can be employed, the only restrictions being that it is water-soluble, can be connected to the ionic block and exhibits the desired features for the surface of the device. The hydrophilic and neutral block of the block polymer can be selected from the group consisting of polyethylene glycol (PEG), polyglyceryl methacrylate (PGMA), polyvinylalcohol, polyacrylamide (PAM), polymethacrylamide, but it is not limited thereto. It is preferred that the hydrophilic and neutral block has protein-resistant and/or anti-fouling properties. More preferably, the hydrophilic and neutral block is a polyethylene glycol (PEG) or a polyacrylamide (PAM), or a combination thereof.

The hydrophilic and neutral block of the first polymer preferably has a molecular weight of less than 10,000 K, more preferably less than 5,000 K, most preferably less than 1,000 K.

The second polymer according to the invention can be a homopolymer or a block polymer. In case the second polymer is a homopolymer, it is a polyelectrolyte. If the second polymer is a block polymer, it comprises at least an ionic block. The polyelectrolyte or the ionic block can be either cationically or anionically chargeable, wherein it is charged oppositely to the ionic block of the first polymer. If the ionic block of the first polymer is a polycation, than the second polymer has an overall anionic character, and if the ionic block of the first polymer is a polyanion, than the second polymer has an overall cationic character.

The second polymer according to the invention can be a homopolymer or a block polymer. It can be chosen from the same group of polyanions and polycations as the ionic block of the first polymer, but is not limited to this list. In principle, any ionic block can be used in the preparation of the complex coacervate core micelles according to the invention, the only restraint being that it is oppositely chargeable to the ionic block of the first polymer. Most preferably, the second polymer is selected from the group of polyacrylic acid (PAA), polymethacrylic acid (PMA), poly(dimethylamino ethylmethacrylate) (PAMA) and poly(N-alkyl-4-vinylpyridinium) (PVP).

In case the second polymer is a homopolymer, it preferably consists of at least 50, more preferably at least 200, and most preferably at least 500 monomeric units. The homopolymer has a molecular weight of preferably less than 100,000 K, more preferably less than 50,000 K, most preferably less than 10,000 K.

In case the second polymer is a block polymer, the ionic block preferably consists of at least 50, more preferably at least 200, and most preferably at least 500 monomeric units. The ionic block of the second polymer has a molecular weight of preferably less than 100,000 K, more preferably less than 50,000 K, most preferably less than 10,000 K.

In case the second polymer is a block polymer, the second block can be a neutral block that is the same or different than the second block of the first polymer. In principle any monomer can be applied, the only restrictions being that a block of these monomers is water-soluble, can be connected to the ionic block and exhibits the desired features for the surface of the device. It is preferred that the hydrophilic and neutral block has protein-resistant and/or anti-fouling properties. Most preferably, the hydrophilic and neutral block is a polyethylene glycol, a polyglyceryl methacrylate or a polyacrylamide, or a combination thereof.

It is an object of the invention to provide with a use of a coating composition for the reduction or prevention of protein adsorption and/or anti-fouling. It is preferred to use the coating composition according to the invention to coat at least one surface of a device.

The surface properties could be of any nature, either hydrophobic or hydrophilic, negatively or positively charged. Without any limitation thereto, a device or apparatus made of metal, metal alloys, ceramics, glasses, silica, wood and polymeric materials and the like may be coated according to this invention.

It is preferred to use the coating composition according to the invention for the coating of a biomedical device, including a wide variety of devices used in the biological, medical or personal care industries, especially those requiring contact with blood or other protein-containing fluids, or requiring contact with tissues. Such applications may be found in externally used artificial organs or extracorpeal therapeutic devices such as, for example, kidney dialysis and hemoperfusion devices as well as implantable or partially implantable artificial organs or devices such as vascular access devices, insulin pump tubing, urinary or venous catheters, etc. In addition, other portions of artificial organ devices may be coated. In an implantable device, for example, the entire external surface area may be coated to increase the device's biocompatibility. All internal blood-contacting portions of a device may be coated to reduce protein binding, thereby reducing or eliminating thrombogenicity.

Other medical devices may be coated, as may various types of labware which is used in conjunction with tissue or cell cultures, protein-containing fluids such as blood or serum, or the like. This would include, but not be limited to, assay plates, supports or membranes, glassware, cell culture or bioreactor devices or assemblies, tubing for blood transfer, blood cell storage bags, filters, pharmaceutical manufacturing and packaging, protein isolation, preparation and purification devices or systems, etc. In addition, woven or non-woven cloth or cloth-like materials used in laboratory or medical settings may be coated or impregnated with the polymers of this invention to increase resistance to protein binding, thereby reducing staining from protein sources. Coated articles prepared according to this invention will be particularly useful for reusable systems, devices, etc., in order to minimise cross-contamination and to facilitate protein removal by washing.

It is further an object of the invention to provide with a process for coating a device, said process comprising:

(i) mixing at least a first and a second polymer in such amounts that the resulting mixture has a fraction of the total number of cationic polymeric groups over the total number of charged groups in the range of 0.2 to 0.8, wherein the first and the second polymer are oppositely charged and wherein the first polymer is a block polymer comprising at least a hydrophilic and neutral block; and

(ii) bringing the resulting mixture in contact with the surface of a device,

wherein the salt concentration in both steps is less than 1 M, more preferably less than 0.2 M and most preferably less than 0.05 M.

Upon mixing of the first and the second polymer in step (i), the ionic groups of the first polymer and the oppositely charged ionic groups on the second polymer induce complex coacervation, leading to a soluble complex without excess charge, as the charges are all restricted to the complex domain that is formed by the coacervate core. The hydrophilic and neutral block(s) form(s) the corona. The size of the core is determined by the amount of charged groups, whereas the size of the corona is entirely dependent on the amount of neutral monomers. The polymeric micelles according to the invention are self-assemblies that are not induced by amphiphilicity or solvent selectivity, and their constituents all exhibit excellent water solubility.

It is thought that the driving forces for this type of phase separation are the entropy gain connected with the liberation of small counterions that were initially confined by the electric field of the participating ionic blocks and the decrease in the electrostatic energy of the ionic blocks due to a more efficient screening of the charges.

The self-assemblies according to the invention lack a CMC. The formation of complex coacervate core micelles is therefore not restricted to the length ratio of the chargeable and neutral blocks and does not have a minimal concentration in which the constituents need to be supplied with.

However, it is found that the formation of the complex coacervate core micelles is rather dependent on the total number of anionically and cationically chargeable groups. In order to define the conditions to work the invention, the composition is not expressed in terms of the fraction of monomeric units, but as the fraction of cationic groups

$$f^+ = \frac{\text{number of cationic groups}}{\text{total number of charged groups}}$$

or the fraction of anionic groups f = 1- f⁺.

There is a limiting range in f⁺ in which self-assembly into polymeric micelles takes place, around the so-called preferred micellar composition (PMC), which relates to the relative amounts of cationic and anionic groups in the composition. Outside this range no complex coacervate core micelles are formed. The PMC does not form a lower limit like the CMC known from amphiphilic micelles, but rather an optimum in a range with a lower and an upper limit. The PMC is found to be at f⁺ = 0.5, when the total amount of positively charged groups more or less equals the total amount of negatively charged groups in the composition. It is preferred that f⁺ is in the range from 0.2 to 0.8, with more preference from 0.3 to 0.7 and with most preference from 0.4 to 0.6.

In an embodiment of the invention the polymeric micelle comprises more than one of the first polymer and more than one of the second polymer, wherein the relative amounts are governed by the above-mentioned restriction on f⁺. The so-called aggregation number relates to the number of polymers that are involved in micellisation.

The aggregation number of the micelles decreases strongly with increasing length of the corona block resulting in a small increase in micellar radius. Therefore the ratio of the core and corona block sizes are reflected in the thickness of the complex coacervate layer attached to the surface and the layer of neutral polymer brushes. Core and corona block lengths are parameters that a person skilled in the art can vary to tune the coating properties to the required application, i.e. binding strength and e.g. antifouling properties respectively.

The micellisation occurrence is determined by (i) the block length ratio; (ii) the total block length of the block polymer; (iii) the chemical structure (i.e. the hydrophilicity) of the corona monomers; and (iv) the molecular weight and type of ionic groups of the second polymer. Stable micelles are only formed if the length of the ionic block and the hydrophilic and neutral block on the block polymer are appropriate.

If the neutral block is too short compared with the charged parts, the system substantially degrades to a mixture of oppositely charged polyelectrolytes and leads to macroscopic phase separation rather than the formation of micelles. However, it is found that too high a ratio of the amount of hydrophilic and neutral monomers over the amount of charged groups does also not lead to the micelles according to the invention. It is therefore preferred to keep the ratio of the number of hydrophilic and neutral monomers over the number of charged groups on the first block polymer in the range of 1 to 10, with more preference in a range of 2 to 9, and with most preference in the range of 3 and 8. If the ratio exceeds 3 precipitation can be avoided completely. This ratio also relates to the hydrophilicity of the hydrophilic and neutral block: When the hydrophilic properties improve, a smaller ratio suffices for micellisation.

In one embodiment of the invention it is preferred to have the ratio in the range of 3 to 10 if the number of charged groups involved in complexation is less than 14, preferably less than 35.

As mentioned above, it is found that in the case of very asymmetric block length ratios, where the corona is much longer than the core block, no micelles are formed. For the invention to work it is therefore preferred that the second polymer has at least 20, more preferably at least 100, and most preferably at least 500 charged monomeric units.

It is further found that complex coacervation is entirely suppressed if the salt concentration reaches a critical value. If the ionic strength in the solutions containing the polyelectrolytes is too high, no complexation will take place upon mixing. Upon addition of salt to a complex coacervation system, the coacervate phase will redissolve into its separate constituents. It is therefore preferred that during the mixing of step (i) and the coating of step (ii) the salt concentration is less than 1 M, more preferably less than 0.2 M and most preferably less than 0.05 M.

It is further an object of the invention to provide with a coated device obtainable by a process as described above.

It is also an object of the invention to provide with a coated device, in which at least one polymeric micelle is physically bonded to at least one surface of the device. The physical bonding between the micelles and the surface is preferably achieved by adsorption from solution. Preferably the surface is dipped into the solution obtained from step (i) for a time sufficient for the polymeric micelles to adsorb from the solution onto the surface, thereby preferably forming a monolayer. The coating application according to the invention may be accomplished in a number of ways which are known by a person having ordinary skill in the art.

The following examples further illustrate the present invention without limiting the scope of the appended claims.

Example 1

Poly(N-methyl-4-vinylpyridiniumjodide (PVP) was used as obtained from Polymer Source Inc. (Montreal, Canada). 0.5 g PVP was dissolved in 10 mM aqueous solution of $NaNO_3$ to a concentration of approximately 0.5 g/l. Separately, two batches with poly(acrylic acid)-co-poly(acryl amide) under the name of PAA42-PAM97 and PAA42-PAM417 as available from Rhodia Chimie (Aubervilliers, France) were prepared by dissolution of 0.5 g PAA42-PAM97 and 0.5 g PAA42-PAM417 in 10 mM $NaNO_3$-solutions to concentrations of about 0.5 g/l. and 1.5 g/l. respectively. The fraction of cationic groups in PVP and the fraction of anionic groups in the block polymers PAA42-PAM97 and PAA42-PAM417 were calculated from the numbers as provided by the manufacturers, see table 1. The polydispersities of all polymers were low, typically around 1.05 - 1.1. All other chemicals (salts, basic and acidic solutions) were of analytical grade.

Table 1 - Block lengths and molecular weight of PAA-PAM and PVP

|  | no. of ionic monomers | no. of neutral monomers | MW (g/mol) |
|---|---|---|---|
| PAA42-PAM97 | 42 | 97 | 10,000 |
| PAA42-PAM417 | 42 | 417 | 33,000 |
| PVP | 209 |  | 56,000 |

The formation of complex coacervate core micelles was now studied by dynamic light scattering upon mixing the PAA42-PAM97- and PAA42-PAM417-batches of various compositions with the PVP-solution in a light scattering cell. Dynamic light scattering was performed with an ALV light scattering instrument equipped with a 400 mW argon ion laser tuned at a wavelength of 514 nm. For each mixture the autocorrelation function was recorded, which arises from the Brownian motion of particles in a liquid, which in case of the detection of particles yielded a diffusion coefficient, that was converted by the Stokes-Einstein equation into a hydrodynamic particle radius.

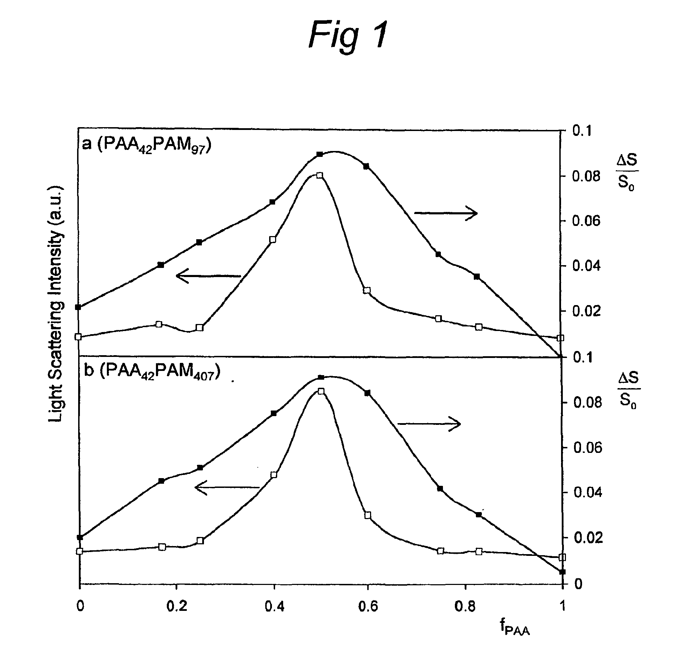

The light scattering results were plotted in figure 1 (open squares) for mixtures of PAA42-PAM97 with PVP (top) and PAA42-PAM417 with PVP (bottom) as a function of the fraction f of anionic PAA-groups over the total number of charged groups (from both PAA and PVP). Along the y-axis the light scattering data were plotted as measured intensities. Only at approximately f = 0.5 particles were detected.

For both batches of block polymer the maximum size was reached when f is about half, meaning that the amount of anionic and cationic groups were roughly equal. The hydrodynamic radii at these concentrations were approximately 20 and 25 nm with PAA42-PAM97 and PAA42-PAM417, respectively.

Example 2

The titration experiment of example 1 was repeated with the batches of 10 mM $NaNO_3$ solutions of PAA42-PAM97, PAA42-PAM417 and PVP as prepared therein. Now the adsorption from these solutions onto a hydrophilic surface was monitored by reflectometry as a function of the fraction of anionic groups. As the surface a silicon wafer (Aurel GmbH, Germany) was applied, carrying an oxide-layer of about 73 nm (as determined by ellipsometry). The pH in these solutions was adjusted to pH 7 using NaOH and $HNO_3$ solutions. At this pH the silica surface is negatively charged.

The adsorption conditions and the conversion of raw reflectometry data in to the adsorbed amount are described in "Reflectometry as a tool for adsorption studies", Adv. Colloid Interface Sci. 1994, vol. 50, p. 79 - 101. The base reflectivity signal $S_0$ was first determined flushing 10 mM $NaNO_3$ through the cell in which the monitored surface was exposed to the solution that was pumped through. The change in reflectivity upon switching to a solution containing a particular mixture of PAA-PAM and PVP was followed until a stable signal $S$ was reached again, corresponding to maximal adsorption. The reflectivity over the reflectivity before addition of the adsorbate $S/S_0$, which is linearly proportional with the adsorbed amount, was determined for different amounts of anionic groups in the solution and plotted together with the light scattering data in figure 1 (filled squares).

The results were in agreement with the previous example, showing that maximal adsorption was reached at roughly equal amounts of PAA- and PVP-groups in the composition. The maximum values corresponded to 3 mg/m$^2$ for both batches. The pure components PAA42-PAM97 and PAA42-PAM417 barely adsorbed on the silica surface (f = 1), and the adsorption of pure PVP (f = 0) was also far behind the levels reached when the fractions of anionic and cationic groups in the solution were comparable.

When exposing the layers to background solvent again, i.e. 10 mM $NaNO_3$ solution, only a few percent of the adsorbed mass was rinsed from the surface. After this quick desorption step, a stable plateau value was obtained again. When the layers were again exposed to the micellar solutions, the adsorbed mass returned to its original plateau value.

Upon exposure to 1 M NaNO$_3$ solution, the adsorbed mass decreased to approximately 80% of the original plateau value, corresponding to a maximal adsorbed amount of 3 mg/m$^2$ for PAA42-PAM97 and PVP, with f = 0.5.

Example 3

0.5 g PAA42-PAM97 and 0.5 g PVP were dissolved in 2 1 of a 0.10 mM NaNO$_3$-solution, yielding f = 0.5. The pH was adjusted to 7 using NaOH and HNO$_3$ solutions. The polydispersities of the polymers were low, typically around 1.05 - 1.1. The chemicals (salts, basic and acidic solutions) were of analytical grade.

Adsorption from these solutions was according to example 2, but now applying a polystyrene coated silica surface layer thickness 66 nm. The molecular weight of the polystyrene, available by the name of P630-St from Polymer Source Inc. (Montreal, Canada) was 850 K. The robustness of the adsorbed layer is tested by exposure to solvent and 1 M NaNO$_3$-solution.

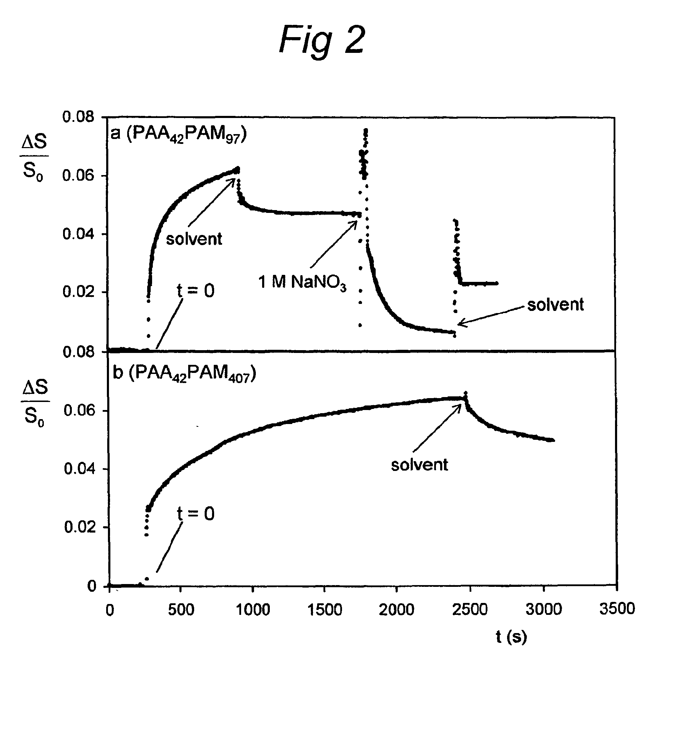

The evolution of the relative adsorption signals $S/S_0$ is shown in figure 2, 0.1 $S/S_0$-unit corresponding with 4 mg/m$^2$. Figure 2 shows that the micelles also adsorbed onto this hydrophobic surface at 0.01 M NaNO$_3$. The exposure to solvent eroded the layers to about two-third of the original signal.

With the addition of 1 M NaNO$_3$-solution the layer eroded even further. The large fluctuations in the signal resulted from large differences in refractive index between 10 mM and 1 M NaNO$_3$, that created a lot of scattering when the liquids were mixed in the reflectometry cell. After a short time, however, a steady signal was obtained again, yielding a plateau value at roughly one-third of the primary adsorption plateau value. Therefore both solvent and 1 M NaNO$_3$ were not able to completely erode the layer.

Example 4

The adsorption of lysozyme on a silica surface, either uncoated or coated with solutions of PAA42-PAM97 / PVP and PAA42-PAM417 / PVP with f = 0.5, was studied using reflectometry according to example 2. Thereto lysozyme from chicken egg white [12650-88-3], obtained from Sigma (L6876), lot. 51K7028, was dissolved in 10 mM NaNO$_3$-solution to a concentration of 100 ppm. The pH was set at about 7 with NaOH solution.

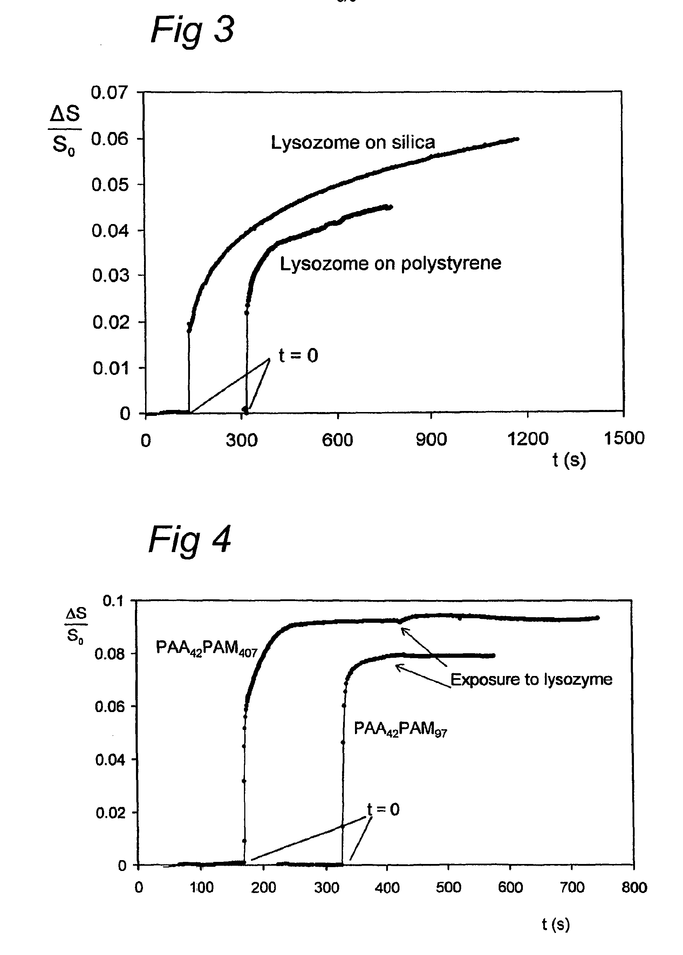

Figure 3 shows the adsorption of lysozyme directly onto a bare silica surface in terms of relative reflectivity $S/S_0$, and figure 4 presents the evolution of the adsorption from the same solution, but now only after coating of the silica surface with either a f = 0.5 solution of PAA42-PAM97 / PVP or PAA42-PAM417 / PVP, as described in example 2. After reaching steady adsorption and before lysozyme addition the reflectometry cell was first flushed with background solvent (10 mM NaNO$_3$) until a stable background signal was obtained.

From the figures 3 and 4 it can be concluded that the adsorption of lysozyme on this hydrophilic surface was prohibited by the layers of polymeric micelles of PAA-PAM and PVP.

Example 5

The variation of the corona block length was investigated by mixing diblock copolymers of polyacrylic acid (PAA) and polyacrylamide (PAM) with a poly-(dimethylamino ethylmethacrylate) (PAMA) homopolymer, that was synthesized according to the method described in Hoogeveen et al. "Novel water-soluble block copolymers of dimethylaminoethyl methacrylate and dihydroxypropyl methacrylate", Macromolecular Chemical Physics, 1996, vol. 197, pages 2553 - 2564. The length of the neutral polyacrylamide-block in the diblock copolymer was an adjustable parameter.

The different batches of PAA-PAM were used as obtained from Rhodia Chimie (Aubervilliers, France) in a concentration of 2.7 mM of PAA-monomers in 30 mM NaNO$_3$. These concentrations were determined on the basis of the specifications given by the manufacturer (see table 2). The polydispersities of all polymers were low, typically around 1.05 - 1.1, the chemicals are of analytical grade.

These diblock copolymers were titrated with PAMA according to example 1. The obtained aqueous mixtures, characterised by the fraction of PAMA-monomers $f_{PAMA}$, were studied with dynamic light scattering.

When $f_{PAMA}$ was between 0.3 to 0.6, particles with hydrodynamic radii of 20 to 40 nm were detected, indicating the formation of complex coacervate core micelles. The measured radii as a function of the monomeric PAM-units showed a linear dependence, yielding the largest particle size for the block of 417 PAM-units.

Table 2 - Variation in block length of neutral block in PAAPAM diblock copolymer

|  | no. of ionic monomers | no. of neutral monomers | MW (g/mol) |
|---|---|---|---|
| PAA42-PAM42 | 42 | 42 | 6,000 |
| PAA42-PAM97 | 42 | 97 | 10,000 |

(continued)

| | no. of ionic monomers | no. of neutral monomers | MW (g/mol) |
|---|---|---|---|
| PAA42-PAM208 | 42 | 208 | 18,000 |
| PAA42-PAM417 | 42 | 417 | 33,000 |
| PAMA | 126 | | |

Example 6

In a DLS-titration study according to example 5 the effect of the molecular weight of the second polymer on the polymeric micelles according the invention was investigated. Thereto, 1 g/l aqueous PAMA35-PGMA105-solution was mixed with aqueous solutions of PMA with a chain length of 1300 and 6900 monomers (respective molecular weight of 113 and 600 K). PAMA35-PGMA105 was synthesized according to the method described in Hoogeveen et al. "Novel water-soluble block copolymers of dimethylaminoethyl methacrylate and dihydroxypropyl methacrylate", Macromolecular Chemical Physics, 1996, vol. 197, pages 2553 - 2564. The concentration of PMA-monomeric units the diblock copolymer was titrated with was 17 mM. The ionic strength was 100 mM NaCl throughout the experiment.
When the fraction of PMA monomers was between approximately 0.4 and 0.6, micelles with hydrodynamic radii of about 15 and 25 nm were detected for PMA1300 and PMA6900, respectively.

Example 7

According to example 6, only using PAMA12-PGMA118 instead of PAMA35-PGMA105 and PAA with 2100 monomeric units (molecular weight 154 K) instead of PMA. The diblock copolymer was dissolved to a concentration of 5 g/l in 100 mM NaNO$_3$, and then titrated with 33 mM PAMA-monomeric units (in 100 mM NaNO$_3$).
Again, when the fraction of PAA monomers was between approximately 0.4 and 0.6, micelles with a radius of about 30 nm size were observed.

**Claims**

1. Use of a coating composition comprising at least one polymeric micelle, wherein the polymeric micelle has a hydrophilic, neutral corona and a complex coacervate core, wherein the complex coacervate core is formed by charge complexation, for rendering at least one surface of a device protein-resistant, for the reduction or prevention of protein adsorption and/or for anti-fouling,
   and wherein:

   - the polymeric micelle comprises at least a first and a second polymer oppositely charged,
   - the first polymer is a block polymer with an ionic block comprising at least 6 chargeable groups, and with a hydrophilic and neutral block.

2. Use according to claim 1, wherein the ionic block is selected from the group of polyacrylic acid, polymethacrylic acid, poly-(dimethylamino ethylmethacrylate) and poly(N-alkyl-4-vinylpyridinium).

3. Use according to any of claims 1 - 2, wherein the hydrophilic and neutral block is a polyethylene glycol, a polyglycerylmethacrylate a polyacrylamide, or a combination thereof.

4. Use according to any of claims 1- 3, wherein the second polymer is a homopolymer or a block polymer.

5. Use according to claim 4, wherein the homopolymer or block polymer is selected from the group of polyacrylic acid, polymethacrylic acid, poly-(dimethylamino ethylmethacrylate) and poly(N-alkyl-4-vinylpyridinium).

6. Use according to any of the preceding claims for the coating of a biomedical device.

7. Process for coating a device, said process comprising:

   (i) mixing at least a first and a second polymer in such amounts that the resulting mixture has a fraction of the total number of cationic polymeric groups over the total number of charged groups in the range of 0.2 to 0.8, wherein the first and the second polymer are oppositely charged and wherein the first polymer is a block polymer with an ionic block comprising at least 6 changeable groups and comprising at least a hydrophilic and neutral block; and

(ii) bringing the resulting mixture under aqueous conditions in contact with the surface of a device,

wherein the salt concentration in both steps is less than 1 M.

8. Process according to claim 7, wherein the ionic block is selected from the group of polyacrylic acid, polymethacrylic acid, poly-(dimethylamino ethylmethacrylate) and poly(N-alkyl-4-vinylpyridinium).

9. Process according to any of claims 7 - 8, wherein the hydrophilic and neutral block is a polyethylene glycol, a polyglycerylmethacrylate or a polyacrylamide, or a combination thereof.

10. Process according to claim 7 - 9, wherein the second polymer is a homopolymer or a block polymer.

11. Process according to claim 10, wherein the homopolymer or block polymer is selected from the group of polyacrylic acid, polymethacrylic acid, poly-(dimethylamino ethylmethacrylate) and poly(N-alkyl-4-vinylpyridinium).

12. Coated device obtainable by the process according to any of claims 7 - 11.

13. Coated device comprising a coated surface, wherein the coated surface comprises at least one polymeric micelle immobilized to the surface of the device, wherein the polymeric micelle has a charged core and a hydrophilic, neutral corona and wherein:

- the polymeric micelle comprises at least a first and a second polymer oppositely charged,
- the first polymer is a block polymer with an ionic block comprising at least 6 chargeable groups, and with a hydrophilic and neutral block.

14. Coated device according to claim 13, wherein the ionic block is selected from the group of polyacrylic acid, polymethacrylic acid, poly-(dimethylamino ethylmethacrylate) and poly(N-alkyl-4-vinylpyridinium).

15. Coated device according to any of claims 13 - 14, wherein the hydrophilic and neutral block is a polyethylene glycol, a polyglycerylmethacrylate or a polyacrylamide, or a combination thereof.

16. Coated device according to any of claims 13 - 15, wherein the second polymer is a homopolymer or a block polymer.

17. Coated device according to claim 16, wherein the homopolymer or block polymer is selected from the group of polyacrylic acid, polymethacrylic acid, poly-(dimethylamino ethylmethacrylate) and poly(N-alkyl-4-vinylpyridinium).

## Patentansprüche

1. Verwendung einer Beschichtungszusammensetzung, umfassend wenigstens eine polymere Micelle, wobei die polymere Micelle eine hydrophile, neutrale Korona und einen komplexen Koazervatkern aufweist, wobei der komplexe Koazervatkern durch Ladungskomplexierung gebildet ist, um wenigstens eine Oberfläche einer Einheit proteinbeständig zu machen zwecks Verringerung oder Verhinderung von Proteinadsorption und/oder zwecks Antifouling, und wobei:

- die polymere Micelle wenigstens ein erstes und ein zweites Polymer umfasst, die entgegengesetzt geladen sind,
- das erste Polymer ein Blockpolymer mit einem ionischen Block, welcher wenigstens 6 ladbare Reste aufweist, und einem hydrophilen und neutralen Block ist.

2. Verwendung gemäß Anspruch 1, wobei der ionische Block ausgewählt ist aus der Gruppe von Polyacrylsäure, Polymethacrylsäure, Poly(dimethylaminoethylmethacrylat) und Poly(N-alkyl-4-vinylpyridin).

3. Verwendung gemäß einem der Ansprüche 1-2, wobei der hydrophile und neutrale Block ein Polyethylenglykol, ein Polyglycerylmethacrylat, ein Polyacrylamid oder eine Kombination davon ist.

**4.** Verwendung gemäß einem der Ansprüche 1-3, wobei das zweite Polymer ein Homopolymer oder ein Blockpolymer ist.

**5.** Verwendung gemäß Anspruch 4, wobei das Homopolymer oder Blockpolymer ausgewählt ist aus der Gruppe von Polyacrylsäure, Polymethacrylsäure, Poly(dimethylaminoethylmethacrylat) und Poly(N-alkyl-4-vinylpyridin).

**6.** Verwendung gemäß einem der vorstehenden Ansprüche zur Beschichtung einer biomedizinischen Einheit.

**7.** Verfahren zum Beschichten einer Einheit, wobei das Verfahren folgendes umfasst:

(i) Mischen von wenigstens einem ersten und einem zweiten Polymer in solchen Mengen, dass das erhaltene Gemisch einen Anteil der Gesamtzahl von kationischen, polymeren Resten gegenüber der Gesamtzahl von geladenen Resten im Bereich von 0,2 bis 0,8 aufweist, wobei das erste und das zweite Polymer entgegengesetzt geladen sind und wobei das erste Polymer ein Blockpolymer mit einem ionischen Block ist, welcher wenigstes 6 ladbare Reste aufweist und wenigstens einen hydrophilen und neutralen Block umfasst; und
(ii) Kontaktieren des so erhaltenen Gemischs unter wässrigen Bedingungen mit der Oberfläche einer Einheit,

wobei die Salzkonzentration bei beiden Schritten weniger als 1 M beträgt.

**8.** Verfahren gemäß Anspruch 7, wobei der ionische Block ausgewählt ist aus der Gruppe von Polyacrylsäure, Polymethacrylsäure, Poly(dimethylaminoethylmethacrylat) und Poly(N-alkyl-4-vinylpyridin).

**9.** Verfahren gemäß einem der Ansprüche 7-8, wobei der hydrophile und neutrale Block ein Polyethylenglykol, ein Polyglycerylmethacrylat, ein Polyacrylamid oder eine Kombination davon ist.

**10.** Verfahren gemäß Anspruch 7-9, wobei das zweite Polymer ein Homopolymer oder ein Blockpolymer ist.

**11.** Verfahren gemäß Anspruch 10, wobei das Homopolymer oder Blockpolymer ausgewählt ist aus der Gruppe von Polyacrylsäure, Polymethacrylsäure, Poly(dimethylaminoethylmethacrylat) und Poly(N-alkyl-4-vinylpyridin).

**12.** Beschichtete Einheit, erhältlich durch das Verfahren gemäß einem der Ansprüche 7-11.

**13.** Beschichtete Einheit, umfassend eine beschichtete Oberfläche, wobei die beschichtete Oberfläche wenigstens eine polymere Micelle umfasst, die an der Oberfläche der Einheit immobilisiert ist, wobei die polymere Micelle einen geladenen Kern und eine hydrophile, neutrale Korona aufweist und wobei

- die polymere Micelle wenigstens ein erstes und ein zweites Polymer umfasst, die entgegengesetzt geladen sind,
- das erste Polymer ein Blockpolymer mit einem ionischen Block, welcher wenigstens 6 ladbare Reste aufweist, und einem hydrophilen und neutralen Block ist.

**14.** Beschichtete Einheit gemäß Anspruch 13, wobei der ionische Block ausgewählt ist aus der Gruppe von Polyacrylsäure, Polymethacrylsäure, Poly(dimethylaminoethylmethacrylat) und Poly(N-alkyl-4-vinylpyridin).

**15.** Beschichtete Einheit gemäß einem der Ansprüche 13-14, wobei der hydrophile und neutrale Block ein Polyethylenglykol, ein Polyglycerylmethacrylat, ein Polyacrylamid oder eine Kombination davon ist.

**16.** Beschichtete Einheit gemäß einem der Ansprüche 13-15, wobei das zweite Polymer ein Homopolymer oder ein Blockpolymer ist.

**17.** Beschichtete Einheit gemäß Anspruch 16, wobei das Homopolymer oder Blockpolymer ausgewählt ist aus der Gruppe von Polyacrylsäure, Polymethacrylsäure, Poly(dimethylaminoethylmethacrylat) und Poly(N-alkyl-4-vinylpyridin).

**Revendications**

**1.** Utilisation d'une composition de revêtement comprenant au moins une micelle polymère, dans laquelle la micelle polymère a une couronne hydrophile neutre et un centre coacervé complexe, le centre coacervé complexe étant formé par complexation de charges, pour rendre au moins une surface d'un dispositif résistant aux protéines, pour la réduction ou la prévention de l'adsorption des protéines et/ou pour la prévention des salissures, et dans laquelle :

- la micelle polymère comprend au moins un premier et un second polymère de charges opposées,
- le premier polymère est un polymère séquencé contenant une séquence ionique, qui comprend au moins 6 groupes chargeables, et contenant une séquence hydrophile et neutre.

**2.** Utilisation selon la revendication 1, dans laquelle la séquence ionique est choisie dans le groupe de l'acide polyacrylique, l'acide polyméthacrylique, le poly-(diméthylamino-éthylméthacrylate) et le poly(N-alkyl-4-vinylpyridinium).

**3.** Utilisation selon l'une quelconque des revendications 1 à 2, dans laquelle la séquence hydrophile et neutre est un polyéthylène glycol, un méthacrylate de polyglycéryle, un polyacrylamide ou une de leurs combinaisons.

**4.** Utilisation selon l'une quelconque des revendications 1 à 3, dans laquelle le second polymère est un homopolymère ou un polymère séquencé.

**5.** Utilisation selon la revendication 4, dans laquelle l'homopolymère ou le polymère séquencé est choisi dans le groupe de l'acide polyacrylique, l'acide polyméthacrylique, le poly-(diméthylamino-éthylméthacrylate) et le poly(N-alkyl-4-vinylpyridinium).

**6.** Utilisation selon l'une quelconque des revendications précédentes, pour le revêtement d'un dispositif biomédical.

**7.** Procédé de revêtement d'un dispositif, ledit procédé comprenant les étapes consistant à :

(i) mélanger au moins un premier et un second polymère en quantités telles que le mélange résultant ait une fraction du nombre total de groupes polymères cationiques sur le nombre total de groupes chargés dans la plage allant de 0,2 à 0, 8, le premier et le second polymère étant de charges opposées et le premier polymère étant un polymère séquencé contenant une séquence ionique, qui comprend au moins 6 groupes chargeables, et contenant au moins une séquence hydrophile et neutre ; et
(ii) mettre le mélange résultant en conditions aqueuses en contact avec la surface d'un dispositif,

dans lequel la concentration en sel dans les deux étapes est inférieure à 1 M.

**8.** Procédé selon la revendication 7, dans lequel la séquence ionique est choisie dans le groupe de l'acide polyacrylique, l'acide polyméthacrylique, le poly-(diméthylamino-éthylméthacrylate) et le poly(N-alkyl-4-vinylpyridinium).

**9.** Procédé selon l'une quelconque des revendications 7 à 8, dans lequel la séquence hydrophile et neutre est un polyéthylène glycol, un méthacrylate de polyglycéryle ou un polyacrylamide ou une de leurs combinaisons.

**10.** Procédé selon les revendications 7 à 9, dans lequel le second polymère est un homopolymère ou un polymère séquencé.

**11.** Procédé selon la revendication 10, dans lequel l'homopolymère ou le polymère séquencé est choisi dans le groupe de l'acide polyacrylique, l'acide polyméthacrylique, le poly-(diméthylamino-éthylméthacrylate) et le poly(N-alkyl-4-vinylpyridinium).

**12.** Dispositif enduit, pouvant être obtenu par le procédé selon l'une quelconque des revendications 7 à 11.

**13.** Dispositif enduit comprenant une surface enduite, dans lequel la surface enduite comprend au moins une micelle polymère immobilisée à la surface du dispositif, la micelle polymère ayant un centre chargé et une couronnne hydrophile neutre, et dans lequel :

- la micelle polymère comprend au moins un premier et un second polymère de charges opposées,
- le premier polymère est un polymère séquencé contenant une séquence ionique, qui comprend au moins 6 groupes chargeables, et contenant une séquence hydrophile et neutre.

**14.** Dispositif enduit selon la revendication 13, dans lequel la séquence ionique est choisie dans le groupe de l'acide polyacrylique, l'acide polyméthacrylique, le poly-(diméthylamino-éthylméthacrylate) et le poly(N-alkyl-4-vinylpyridinium).

15. Dispositif enduit selon l'une quelconque des revendications 13 à 14, dans lequel la séquence hydrophile et neutre est un polyéthylène glycol, un méthacrylate de polyglycéryle ou un polyacrylamide ou une de leurs combinaisons.

16. Dispositif enduit selon l'une quelconque des revendications 13 à 15, dans lequel le second polymère est un homopolymère ou un polymère séquencé.

17. Dispositif enduit selon la revendication 16, dans lequel l'homopolymère ou le polymère séquencé est choisi dans le groupe de l'acide polyacrylique, l'acide polyméthacrylique, le poly-(diméthylamino-éthylméthacrylate) et le poly(N-alkyl-4-vinylpyridinium).

# Fig 1

## Fig 2

## Fig 3

## Fig 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0132230 A

### Non-patent literature cited in the description

- Reflectometry as a tool for adsorption studies. *Adv. Colloid Interface Sci.,* 1994, vol. 50, 79-101

- **HOOGEVEEN et al.** Novel water-soluble block copolymers of dimethylaminoethyl methacrylate and dihydroxypropyl methacrylate. *Macromolecular Chemical Physics,* 1996, vol. 197, 2553-2564